# EUROPÄISCHE PATENTSCHRIFT

(11) **EP 1 776 085 B1**
(45) Veröffentlichungstag und Bekanntmachung des Hinweises auf die Patenterteilung: **28.04.2010**
(21) Anmeldenummer: 05762372.0
(22) Anmeldetag: 26.07.2005
(51) Int. Cl.: A61K 8/18, A61Q 5/08, A61Q 5/10

(54) **ALKALISIERUNGSMITTEL-HALTIGES MITTEL ZUM AUFHELLEN UND/ODER FÄRBEN VON KERATINHALTIGEN FASERN**
AGENT CONTAINING AN ALKALISATION AGENT FOR LIGHTENING AND/OR COLOURING KERATIN FIBRES
AGENTS CONTENANT DES AGENTS D'ALCALISATION DESTINES A ECLAIRCIR ET/OU COLORER DES FIBRES KERATINIQUES

(30) Priorität: 10.08.2004 DE 102004038991
(43) Veröffentlichungstag der Anmeldung: 25.04.2007
(73) Patentinhaber: Henkel AG & Co. KGaA, 40589 Düsseldorf (DE)
(72) Erfinder: HÖFFKES, Horst, 40595 Düsseldorf (DE); SEILER, Martina, 47228 Duisburg (DE); PAULI, Kristin, 42119 Wuppertal (DE)
(86) Internationale Anmeldenummer: PCT/EP2005/008091
(87) Internationale Veröffentlichungsnummer: WO 2006/018099

(56) Entgegenhaltungen:
- EP-A- 0 583 767
- WO-A-03/022232
- US-A- 5 786 313
- US-A- 5 961 663
- US-B1- 6 585 987
- SCHLOSSMANN M.: 'The chemistry and manufacture of cosmetics. Vol.II- Formulating, edition 3.', 2000, ALLURED, USA * Seite 405 - Seite 406 *

## Beschreibung

Die Erfindung betrifft Mittel zum Aufhellen und/oder Färben von keratinhaltigen Fasern, insbesondere Blondiermittel für menschliche Haare.

Die Veränderung von Form und Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepaßt werden. Dabei können Dauerwell- und andere die Haarform verändernde Verfahren nahezu unabhängig vom Typ der zu behandelnden Haare eingesetzt werden. Dagegen sind Färbe- und insbesondere Blondierverfahren auf bestimmte Ausgangshaarfarben begrenzt. So eignen sich für aufhellende Verfahren, die sogenannten Blondierverfahren, im wesentlichen nur dunkelblonde oder hellere Haare. Die Grundlagen der Blondierverfahren sind dem Fachmann bekannt und können in einschlägigen Monographien, z.B. von Kh. Schrader, Grundlagen und Rezepturen der Kosmetika, 2. Auflage, 1989, Dr. Alfred Hüthig Verlag, Heidelberg, oder W. Umbach (Hrg.), Kosmetik, 2. Auflage, 1995, Georg Thieme Verlag, Stuttgart, New York, nachgelesen werden.

So werden üblicherweise feste oder pastenformige Zubereitungen mit festen Oxidationsmitteln unmittelbar vor der Anwendung mit einer verdünnten Wasserstoffperoxidlösung vermischt. Diese Mischung wird dann auf das Haar aufgebracht und nach einer bestimmten Einwirkzeit wieder ausgespült.

Die genannten Zubereitungen, die vor der Anwendung mit einer Wasserstoffperoxidlösung vermischt werden, werden im weiteren als "Blondiermittel" bzw. "Blondierpulver" bezeichnet. Alle aufgeführten Mengenangaben beziehen sich, soweit nicht anders ausgeführt, ausschließlich auf diese Zubereitungen.

Die vorliegende Erfindung bezieht sich auch auf Mittel zum gleichzeitigen Färben und Aufhellen von keratinhaltigen Fasern, insbesondere menschlichen Haaren. Konventionelle Haarfärbemittel bestehen in der Regel aus mindestens einer Entwickler-und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.
Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

WO03/022232A beschreibt Haarfärbemittel, welche direktziehende Farbstoffe und Oxidationsmittel enthalten.

Vor ihrer Anwendung auf menschliches Haar werden Haarfärbemittel mit Oxidationsfarbstoffvorprodukten mit verdünnter wäßriger Wasserstoffperoxid-Lösung vermischt. Die Einwirkungsdauer auf dem Haar zur Erzielung einer vollständigen Ausfärbung liegt zwischen etwa 30 und 40 Minuten. Es ist naheliegend, daß bei den Benutzern dieser Haarfarben ein Bedürfnis besteht, diese Einwirkungszeit zu verringern.

Gleichzeitig besteht auch vielfach der Wunsch, zusammen mit der Haarfärbung auch eine Aufhellung des zu färbenden Haares, die sich auch als erhöhter Glanz bemerkbar macht, zu erreichen.

Sowohl in reinen Blondiermitteln als auch In aufhellenden Färbemitteln besteht das Problem, daß ein Oxidationsmittel, das dem Mittel zugesetzt wird, um ein Anwendungsprodukt zu erhalten, schnell, zersetzt wird. Diese Zersetzungsreaktion wird insbesondere durch Alkallsierungsmittel beschleunigt. Bei Zersetzung von Wasserstoffperoxid tritt eine Gasentwicklung ein. Diese kann während der Handhabung der Blondiermittel je nach Intensität ein Gefahrenpotenzial für den Anwender In sich bergen.

Der vorliegenden Erfindung lag die Aufgabe zugrunde, Mittel zum Aufhellen und/oder Färben von Keratinfasern bereitzustellen, die eine bessere Stabilität von Oxidationsmitteln, die dem Mittel vor der Anwendung zugesetzt werden, ermöglichen. Insbesondere Wasserstoffperoxid sollte dabei stabilisiert werden, ohne daß auf den Einsatz von Alkalisierungsmitteln verzichtet werden muß.

In einer ersten Ausführungsform betrifft die vorliegende Erfindung Mittel zum Aufhellen und/oder Färben von Keratinfasern, insbesondere menschlichen Haaren, enthaltend mindestens ein festes Alkalisierungsmittel, das einen Partikelkern umfaßt, der Alkalisierungsmittel enthält und eine diesen Kern umgebende Hülle aufweist, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Komplexbildner(n) besteht.

EP583767A offenbart staubfreie, partikuläre Haarbehandlungssprays, die durch Verwendung einer inerten Flüssigkeit nur Partikel mit einem Durchmesser von mehr als 40 µm enthalten.

Nachfolgend werden die erfindungsgemäßen Mittel zum Aufhellen und/oder Färben von Keratinfasern auch als "Blondier- bzw. Aufhell- und Färbemittel", als "Blondlermittel", als "Aufhellmittel" oder als "Färbemittel" bezeichnet. Diese Begriffe sind dabei nicht als beschränkend zu verstehen, so daß ein "Blondiermittel" neben der entfärbenden Wirkung durchaus auch zusätzlich färbende Eigenschaften aufweisen kann, während ein "Färbemittel" auch aufhellende Eigenschaften aufweisen kann.

Erfindungsgemäße Mittel enthalten ein Alkalisierungsmittel, welches mit einer Hülle aus Komplexbildnern umgeben ist.

US5,786,313A offenbart Glycin-N,N-diessigsäure-Derivate als Komplexierungsmittel für Ca²⁺-lonen in Textilwaschmitteln und in Mundhygienemitteln. Aus US6,585,987B sind Komplexe von Hyaluronsäure mit Carnitin für die therapeutische und kosmetische Anwendungen bekannt.

Das Alkalisierungsmittel dient zur Einstellung eines alkalischen pH-Wertes der Anwendungsmischung. Erfindungsgemäß können die dem Fachmann die für Blondier- und/oder Färbemittel bekannten, üblichen Alkalisierungsmittel wie Ammonium-, Alkalimetall- und Erdalkalimetallhydroxyde, -carbonate, hydrogencarbonate, - hydroxycarbonate, -sillkate, insbesondere -metasilikate, sowie Alkallphosphate verwendet werden. Auch kurzkettige Amine wie Monoethanolamine, 3-Amino-2-methylpropanol oder alkalisch reagierende Aminosäuren wie Arginin, Omithin und Lysin sind einsetzbar. In einer bevorzugten Ausführungsform enthalten die erfindungsgemäßen Mittel mindestens zwei unterschiedliche Alkalisierungsmittel. Dabei können Mischungen beispielsweise aus einem Metasilikat und einem Hydroxycarbonat bevorzugt sein.

US5,961,663A beschreibt mikrowellen-getrocknete, amorphe Alkalimetallsilikate als Builder In Textilwaschmitteln zur Komplexierung von Ionen, die die Wasserhärte des Waschwassers erhöhen.

Die erfindungsgemäßen Mittel enthalten Alkalisierungsmittel (berechnet als unbeschichtetes Alkalisierungsmittel) bevorzugt In Mengen von 1-30 Gew.-%, insbesondere 2-25 Gew.-%, Jeweils bezogen auf das gesamte Mittel.

Das beschichtete Alkalisierungsmittel, das In den erfindungsgemäßen Mitteln enthalten ist, umfaßt einen Partikelkern, der ein oder mehrere Alkalisierungsmittel enthält und eine diesen Kern umgebende Hülle, wobei die Hülle zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Komplexbildner(n) besteht.

Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-Atomen in der Regel unter Bildung von Chelat-Komplexen reagieren, sind erfindungsgemäß einsetzbar. Die Polymer-gebundenen Liganden der entstehenden Metall-Komplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Komplexierende Gruppen (Liganden) üblicher komplexbildender Polymere sind Iminodiessigsäure-, Hydroxychinolin-, Thioharnstoff-, Guanidin-, Dithiocarbamat-, Hydroxamsäure-, Amidoxim-, Aminophosphorsäure-, (cycl.) Polyamino-, Mercapto-, 1,3-Dicarbonyl- und Kronenether-Reste mit z. T. sehr spezif. Aktivitäten gegenüber Ionen unterschiedlicher Metalle. Basispolymere vieler auch kommerziell bedeutender komplexbildender Polymere sind Polystyrol, Polyacrylate, Polyacrylnitrile, Polyvinylalkohole, Polyvinylpyridine und Polyethylenimine. Auch natürliche Polymere wie Cellulose, Stärke od. Chitin sind komplexbildende Polymere. Darüber hinaus können diese durch polymeranaloge Umwandlungen mit weiteren Ligand-Funktionalitäten versehen werden.

Besonders bevorzugt ist im Rahmen der vorliegenden Erfindung der Einsatz eines oder mehrerer Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine.

Im Rahmen der vorliegenden Erfindung können alle Komplexbildner des Standes der Technik eingesetzt werden. Diese können unterschiedlichen chemischen Gruppen angehören. Vorzugsweise werden einzeln oder im Gemisch miteinander eingesetzt:
a) Polycarbonsäuren, bei denen die Summe der Carboxyl- und gegebenenfalls Hydroxylgruppen mindestens 5 beträgt wie Gluconsäure,
b) stickstoffhaltige Mono- oder Polycarbonsäuren wie Ethylendiamintetraessigsäure (EDTA), N-Hydroxyethylethylendiamintriessigsäure, Diethylentriaminpentaessigsäure, Hydroxyethyliminodiessigsäure, Nitridodiessigsäure-3-propionsäure, Isoserindiessigsäure, N,N-Di-(β-hydroxyethyl)-glycin, N-(1,2-Dicarboxy-2-hydroxyethyl)-gly-cin, N-(1,2-Dicarboxy-2-hydroxyethyl)-asparaginsäure oder Nitrilotriessigsäure (NTA),
c) geminale Diphosphonsäuren wie 1-Hydroxyethan-1,1-diphosphonsäure (HEDP), deren höhere Homologe mit bis zu 8 Kohlenstoffatomen sowie Hydroxy- oder Aminogruppen-haltige Derivate hiervon und 1-Aminoethan-1,1-diphosphonsäure, deren höhere Homologe mit bis zu 8 Kohlenstoffatomen sowie Hydroxy- oder Aminogruppen-haltige Derivate hiervon,
d) Aminophosphonsäuren wie Ethylendiamintetra(methylenphosphonsäure), Di-ethylen-triaminpenta(methylenphosphonsäure) oder Nitrilotri(methylenphosphonsäure),
e) Phosphonopolycarbonsäuren wie 2-Phosphonobutan-1,2,4-tricarbonsäure sowie
f) Cyclodextrine.

Als Polycarbonsäuren a) werden im Rahmen dieser Patentanmeldung Carbonsäuren - auch Monocarbonsäuren- verstanden, bei denen die Summe aus Carboxyl- und den im Molekül enthaltenen Hydroxylgruppen mindestens 5 beträgt. Komplexbildner aus der Gruppe der stickstoffhaltigen Polycarbonsäuren, insbesondere EDTA, sind bevorzugt. Bei den erfindungsgemäß erforderlichen alkalischen pH-Werten der Behandlungslösungen liegen diese Komplexbilner zumindest teilweise als Anionen vor. Es ist unwesentlich, ob sie in Form der Säuren oder in Form von Salzen eingebracht werden. Im Falle des Einsatzes als Salze sind Alkali-, Ammonium- oder Alkylammoniumsalze, insbesondere Natriumsalze, bevorzugt.

Ebenso sind als weitere bevorzugte Komplexbildner polymere Aminodicarbonsäuren, deren Salze oder deren Vorläufersubstanzen zu nennen. Besonders bevorzugt sind Polyasparaginsäuren bzw. deren Salze und Derivate, die neben Cobuilder-Eigenschaften auch eine bleichstabilisierende Wirkung aufweisen.

Weitere geeignete Komplexbildner sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Eine weitere Substanzklasse mit komplexbildenden Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylen-phosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Komplexbildner wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Bedeutung sind. Diese Stoffe werden nachstehend beschrieben.

Erfindungsgemäß bevorzugte Komplexbildner sind Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz.

Erfindungsgemäß bevorzugte Mittel sind **dadurch gekennzeichnet, daß** die Hülle als Komplexbildner Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthält.

Um die Haftung der Hülle aus Komplexbildner(n) auf dem Kern aus Alkalisierungsmittel zu verbessern und/oder die physikalischen Eigenschaften der Hülle positiv zu beeinflussen, kann die Hülle weitere Inhaltsstoffe aufweisen. Bevorzugte erfindungsgemäße Mittel beinhalten beschichtete Alkalisierungsmittel, deren Hülle neben Komplexbildner(n) noch Bindemittel enthält.

Als Bindemittel eignen sich beispielsweise natürliche oder synthetische Polymere. Unter diesen sind beispielsweise Polyethylen- bzw. Polypropylengylcole bevorzugt. Bevorzugt sind auch nicht-ionogene Polymere wie beispielsweise:
- Polyvinylpyrrolidone, wie sie beispielsweise unter der Bezeichnung Luviskol^{®} (BASF) vertrieben werden. Polyvinylpyrrolidone [Poly(1-vinyl-2-pyrrolidinone)], Kurzzeichen PVP, sind Polymere der allg. Formel (II) 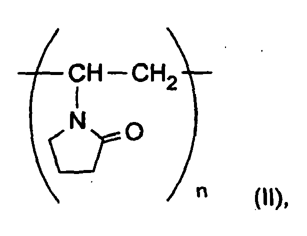 die durch radikalische Polymerisation von 1-Vinylpyrrolidon nach Verfahren der Lösungs- oder Suspensionspolymerisation unter Einsatz von Radikalbildnern (Peroxide, AzoVerbindungen) als Initiatoren hergestellt werden. Die ionische Polymerisation des Monomeren liefert nur Produkte mit niedrigen Molmassen. Handelsübliche Polyvinylpyrrolidone haben Molmassen im Bereich von ca. 2500-750000 g/mol, die über die Angabe der K-Werte charakterisiert werden und - K-Wert-abhängig Glasübergangstemperaturen von 130-175° besitzen. Sie werden als weiße, hygroskopische Pulver oder als wäßrige. Lösungen angeboten. Polyvinylpyrrolidone sind gut löslich in Wasser und einer Vielzahl von organischen Lösungsmitteln (Alkohole, Ketone, Eisessig, Chlorkohlenwasserstoffe, Phenole u.a.).
- VinylpyrrolidonNinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionischen Polymere.

Die Vinylester-Polymere sind aus Vinylestern zugängliche Polymere mit der Gruppierung der Formel (III) 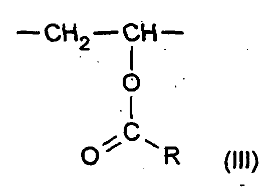 als charakteristischem Grundbaustein der Makromoleküle. Von diesen haben die Vinylacetat-Polymere (R = CH₃) mit Polyvinylacetaten als mit Abstand wichtigsten Vertretern die größte technische Bedeutung.

Die Polymerisation der Vinylester erfolgt radikalisch nach unterschiedlichen Verfahren (Lösungspolymerisation, Suspensionspolymerisation, Emulsionspolymerisation, Substanzpolymerisation.). Copolymere von Vinylacetat mit Vinylpyrrolidon enthalten Monomereinheiten der Formeln (II) und (III)
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden. Celluloseether lassen sich durch die folgende allgemeine Formel beschreiben, 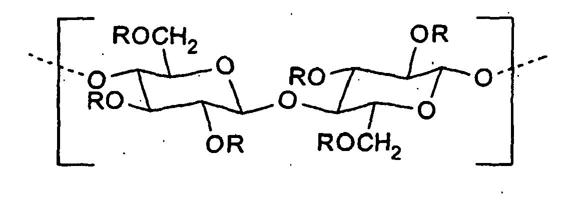 in R für H oder einen Alkyl-, Alkenyl-, Alkinyl-, Aryl- oder Alkylarylrest steht. In bevorzugten Produkten steht mindestens ein R in Formel für -CH₂CH₂CH₂-OH oder - CH₂CH₂-OH. Celluloseether werden technisch durch Veretherung von Alkalicellulose (z.B. mit Ethylenoxid) hergestellt. Celluloseether werden charakterisiert über den durchschnittlichen Substitutionsgrad DS bzw. den molaren Substitutionsgrad MS, die angeben, wie viele Hydroxy-Gruppen einer Anhydroglucose-Einheit der Cellulose mit dem Veretherungsreagens reagiert haben bzw. wie viel mol des Veretherungsreagens im Durchschnitt an eine Anhydroglucose-Einheit angelagert wurden. Hydroxyethylcellulosen sind ab einem DS von ca. 0,6 bzw. einem MS von ca. 1 wasserlöslich. Handelsübliche Hydroxyethyl- bzw. Hydroxypropylcellulosen haben Substitutionsgrade im Bereich von 0,85-1,35 (DS) bzw. 1,5-3 (MS). Hydroxyethyl- und - propylcellulosen werden als gelblich-weiße, geruch- und geschmacklose Pulver in stark unterschiedlichen Polymerisationsgraden vermarktet. Hydroxyethyl- und -propylcellulosen sind in kaltem und heißem Wasser sowie in einigen (wasserhaltigen) organischen Lösungsmitteln löslich, in den meisten (wasserfreien) organischen Lösungsmitteln dagegen unlöslich; ihre wäßrigen Lösungen sind relativ unempfindlich gegenüber Änderungen des pH-Werts oder Elektrolyt-Zusatz.

Polyvinylalkohole, kurz als PVAL bezeichnet, sind Polymere der allgemeinen Struktur

[-CH₂-CH(OH)-]ₙ

die in geringen Anteilen auch Struktureinheiten des Typs

[-CH₂-CH(OH)-CH(OH)-CH₂]

enthalten. Da das entsprechende Monomer, der Vinylalkohol, in freier Form nicht beständig ist, werden Polyvinylalkohole über polymeranaloge Reaktionen durch Hydrolyse, technisch insbesondere aber durch alkalisch katalysierte Umesterung von Polyvinylacetaten mit Alkoholen (vorzugsweise Methanol) in Lösung hergestellt. Durch diese technischen Verfahren sind auch PVAL zugänglich, die einen vorbestimmbaren Restanteil an Acetatgruppen enthalten.

Handelsübliche PVAL (z.B. Mowiol^{®}-Typen der Firma Clariant) kommen als weißgelbliche Pulver oder Granulate mit Polymerisationsgraden im Bereich von ca. 500-2500 (entsprechend Molmassen von ca. 20.000-100.000 g/mol) in den Handel und haben unterschiedliche Hydrolysegrade von 98-99 bzw. 87-89 Mol-%. Sie sind also teilverseifte Polyvinylacetate mit einem Restgehalt an Acetyl-Gruppen von ca. 1-2 bzw. 11-13 Mol%.

Die Wasserlöslichkeit von PVAL kann durch Nachbehandlung mit Aldehyden (Acetalisierung), durch Komplexierung mit Ni- oder Cu-Salzen oder durch Behandlung mit Dichromaten, Borsäure, Borax verringern und so gezielt auf gewünschte Werte eingestellt werden.

Erfindungsgemäß besonders bevorzugte Mittel sind **dadurch gekennzeichnet, daß** die Hülle neben Komplexbildner(n) Bindemittel enthält, wobei als Bindemittel natürliche Polymere, vorzugsweise Cellulose und/oder Stärke sowie deren Derivate, insbesondere Carboxymethylcellulose (CMC) und/oder Hydroxypropylcellulose (HPC) und/oder Hydroxypropylmethylcellulose (HPMC) bevorzugt sind und bevorzugte Mengen der Hülle an Bindemittel 1 bis 50 Gew.-%, vorzugsweise 5 bis 40 Gew.-% und insbesondere 10 bis 30 Gew.-% Bindemittel (jeweils bezogen auf die Beschichtung) betragen.

Die erfindungsgemäßen Blondier- bzw. Aufhell- und Färbemittel enthalten vorzugsweise zusätzlich eine Peroxoverbindung. Die Auswahl dieser Peroxoverbindung unterliegt prinzipiell keinen Beschränkungen. Bevorzugte Peroxoverbindungen sind Wasserstoffperoxid (H₂O₂), beispielsweise in Form einer wäßrigen Lösung oder in Form eines H₂O₂-Adduktes an feste Träger, wobei Harnstoffperhydrat oder Natriumcarbonat-Peroxohydrat ("Natriumpercarbonat") eine besondere Bedeutung besitzen. Neben Wasserstoffperoxid oder an seiner Stelle können auch andere Peroxoverbindungen in den erfindungsgemäßen Mitteln enthalten sein.

übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Kaliumperoxidiphosphat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten.

Bevorzugte erfindungsgemäße Mittel sind daher **dadurch gekennzeichnet, daß** sie zusätzlich eine feste Peroxoverbindung enthalten, die vorzugsweise ausgewählt ist aus Wasserstoffperoxid-Anlagerungsverbindungen an feste Träger, Ammonium- und Alkalimetall-persulfaten und -peroxidisulfaten, wobei besonders bevorzugte erfindungsgemäße Mittel mindestens 2 verschiedene Peroxidisulfate enthalten.

Die Peroxoverbindungen sind in den erfindungsgemäßen Blondier- bzw. Aufhell- und Färbemitteln bevorzugt in Mengen von 2-80 Gew.-%, insbesondere in Mengen von 5-50 Gew.-% enthalten.

Weiterhin hat es sich als vorteilhaft erwiesen, wenn die erfindungsgemäßen Blondiermittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Bevorzugte nichtionogene grenzflächenaktive Stoffe sind
- alkoxylierte Fettalkohole mit 8 bis 22, insbesondere 10 bis 16, Kohlenstoffatomen in der Fettalkylgruppe und 1 bis 30, insbesondere 1 bis 15, Ethylenoxid- und/oder Propylenoxid-Einheiten. Bevorzugte Fettalkylgruppen sind beispielsweise Lauryl-, Myristyl-, Cetyl-, aber auch Stearyl-, Isostearyl- und Oleylgruppen. Besonders bevorzugte Verbindungen dieser Klasse sind beispielsweise Laurylalkohol mit 2 bis 4 Ethylenoxid-Einheiten, Oleyl- und Cetylalkohol mit jeweils 5 bis 10 Ethylenoxideinheiten, Cetyl- und Stearylalkohol sowie deren Mischungen mit 10 bis 30 Ethylenoxideinheiten sowie das Handelsprodukt Aethoxal^{®}B (Henkel), ein Laurylalkohol mit jeweils 5 Ethylenoxid- und Propylenoxideinheiten. Neben den üblichen alkoxylierten Fettalkoholen können auch sogenannte "endgruppenverschlossene" Verbindungen erfindungsgemäß eingesetzt werden. Bei diesen Verbindungen weist die Alkoxygruppe am Ende keine OH-Gruppe auf, sondern ist in Form eines Ethers, insbesondere eines C1-C4-Alkyl-Ethers, "verschlossen". Ein Beispiel für eine solche. Verbindung ist das Handelsprodukt Dehypon^{®}LT 054, ein C₁₂₋₁₈-Fettalkoholol + 4,5 Ethylenoxid-butylether.
- alkoxylierte Fettsäuren mit 8 bis 22, insbesondere 10 bis 16, Kohlenstoffatomen in der Fettsäuregruppe und 1 bis 30, insbesondere 1 bis 15, Ethylenoxid- und/oder Propylenoxid-Einheiten. Bevorzugte Fettsäuren sind beispielsweise Laurin-, Myristin-, Palmitin-, Stearin-, Isostearin- und Ölsäure.
- alkoxylierte, bevorzugt propoxylierte und insbesondere ethoxylierte, Mono-, Di- und Triglyceride. Beispiele für bevorzugte Verbindungen sind Glycerinmonolaurat + 20 Ethylenoxid und Glycerinmonostearat + 20 Ethylenoxid.
- Polyglycerinester und alkoxylierte Polyglycerinester. Bevorzugte Verbindungen dieser Klasse sind beispielsweise Poly(3)glycerindiisostearat (Handelsprodukt: Lameform^{®}TGI (Henkel)) und Poly(2)glycerinpolyhydroxystearat (Handelsprodukt: Dehymuls^{®}PGPH (Henkel)).
- Sorbitan-Fettsäureester und alkoxylierte Sorbitan- Fettsäureester wie beispielsweise Sorbitanmonolaurat und Sorbitanmonolaurat + 20 Ethylenoxid (EO).
- Alkylphenole und Alkylphenolalkoxylate mit 6 bis 21, insbesondere 6 bis 15, Kohlenstoffatomen in der Alkylkette und 0 bis 30 Ethylenoxid- und/oder PropylenoxidEinheiten. Bevorzugte Vertreter dieser Klasse sind beipielsweise Nonylphenol + 4 EO, Nonylphenol + 9 EO, Octylphenol + 3 EO und Octylphenol + 8 EO.

Besonders bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.

Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Blondiermittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tenside auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigen C8-C22-Carbonsäuren wie Ölsäure, Stearinsäure, Isostearinsäure und Palmitinsäure.

Diese anionischen Tenside sollten bevorzugt in fester, insbesondere Pulverform vorliegen. Ganz besonders bevorzugt sind dabei bei Raumtemperatur feste Seifen, insbesondere Natriumstearat. Diese liegen bevorzugt in Mengen von 5 bis 20 Gew.-%, insbesondere 10 bis 15 Gew.-.%, vor.

Als nichtionische Tenside eignen sich insbesondere C8-C22-Alkylmono- und oligoglycoside und deren ethoxylierte Analoga. Insbesondere die nichtethoxylierten Verbindungen haben sich als besonders geeignet erwiesen.

Beispiele für die in den erfindungsgemäßen Haarbehandlungsmittein verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie das unter der Bezeichnung Dehyquart^{®}F 75 in Abmischung mit Cetearylalkohle erhältliche Distearoylethylhydroxyethylammoniummethosulfat.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether und andere, als Feststoff stabile bzw. im Handel erhältliche Verbindungen,
- zwitterionische und amphotere Polymere, die als Feststoffe stabil bzw. bevorzugt als Handelsprodukte erhältlich sind,
- anionische Polymere wie beispielsweise Polyacrylsäuren, vernetzte Polyacrylsäuren und Vinylacetat/Crotonsäure-Copolymere, sofern diese als Feststoffe stabil bzw. bevorzugt im Handel erhältlich sind,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol.
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Farbstoffe zum Einfärben der Zubereitungen,
- Wirkstoffe wie Panthenol, Pantothensäure, Allantoin, Pyrrolidoncarbonsäuren und deren Salze,
- Cholesterin,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Carbonate, Hydrogencarbonate, Guanidine, Harnstoffe sowie primäre, sekundäre und tertiäre Phosphate,

Die Auswahl dieser weiteren Stoffe wird der Fachmann gemäß der gewünschten Eigenschaften der Mittel treffen.

Die erfindungsgemäßen Mittel können zusätzlich ein oder mehrere Farbstoffvorprodukte enthalten.

Erfindungsgemäße Mittel, die zusätzlich mindestens ein Oxidationsfarbstoffvorprodukt vom Entwicklertyp und/gegebenenfalls mindestens ein Oxidationsfarbstoffvorprodukt vom Kupplertyp enthalten, sind dabei bevorzugt.

Hinsichtlich der in den erfindungsgemäßen Blondiermitteln einsetzbaren weiteren Farbstoffvorprodukte unterliegt die vorliegende Erfindung keinerlei Einschränkungen. Die erfindungsgemäßen Blondiermittel können als weitere Farbstoffvorprodukte
- Oxidationsfarbstoffvorprodukte vom Entwickler- und/oder Kuppler-Typ, und
- Vorstufen naturanaloger Farbstoffe, wie Indol-und Indolin-Derivate,
sowie Mischungen von Vertretern dieser Gruppen enthalten.

In einer ersten bevorzugten Ausführungsform enthält das Blondiermittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 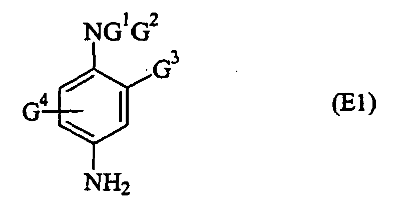 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin. 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenyfendiamin, p-Toluylendiamin, 2-(β-Hydroxyethyl)-p-phenylendiamin, 2-(α,β-Dihydroxyethyl)-p-phenytendiamin und N,N-Bis-(β-hydroxyethyl)-p-phenytendiamin.

Es kann erfindungsgemäß weiterhin bevorzugt sein, als Entwicklerkömponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Mitteln gemäß der Erfindung verwendet werden können, kann man insbesondere die Verbindungen nennen, die der folgenden Formel (E2) entsprechen, sowie ihre physiologisch verträglichen Salze: 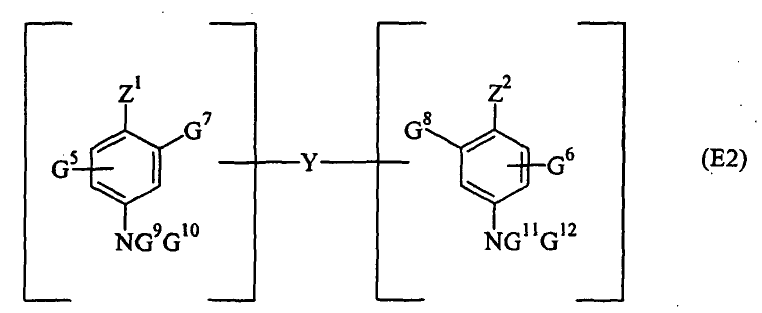 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituierte ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten, der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N.N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonderes bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 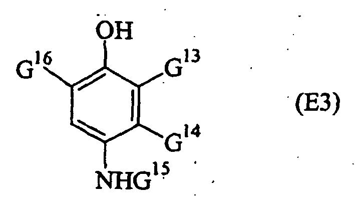 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenql, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(D-hydroxyethoxy)-phenol, 4-Amino-2-methylphenol, 4-Amino-2-hydroxymethylphenot, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwickterkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-l-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 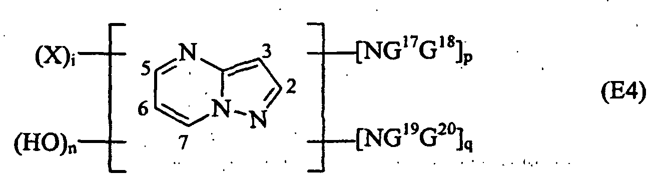 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁₋ bis C₄-Hydroxyalkylrest; einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)aminoalkylrest, einen Aminorest, einen C₁- bis C₄-Alkyl- oder Di-(C₁- bis C₄-hydroxyalkyl)aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formen (E4) eine Hydroxygruppe an einer der Positionen 2, 5 oder 7 des Ringsystems enthält, besteht ein tautomeres Gleichgewicht, das zum Beispiel im folgenden Schema dargestellt wird:

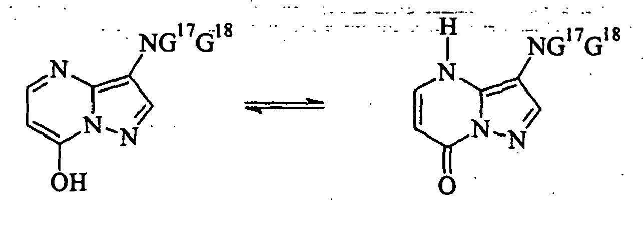

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)amino]-ethanol;
- 2-[(7-Aminopyrazol-[1,5-a]-pyrimidin-3-yl)-(2-hydroxy-ethyl)amino]-ethanol;
- 5,6-Dimethylpyrazol-[1,5-a]-pyrimidin-3,7-diamin;
- 2,6-Dimethytpyrazol-[1,5-a]-pyrimidin-3,7-dianmin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1.5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Mittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-pcopan, 1-Methoxy-2-amino-4-(2'-hydroxyethylamino)benzol, 1,3-Bis-(2',4'-diaminophenyf)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthatin, 1,7-Dihydroxynaphthatin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chihoxalinderivate wie beispielsweise 6-Methyl-,2,3,4-tetrahydrochinoxalin;
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Mittel mindestens ein Indol- und/oder Indolinderivat.

Besonders gut als Vorstufen naturanaloger Haarfarbstoffe geeignet sind Derivate des 5,6-Dihydroxyindolins der Formel (IIIa), 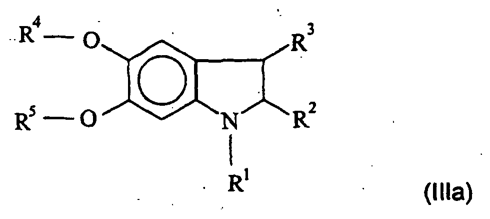 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, . N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIIb), 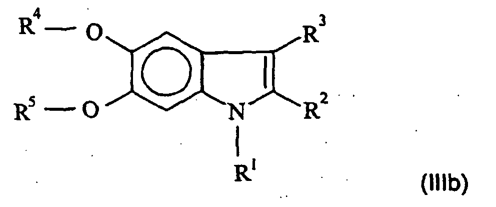 in der unabhängig voneinander
- R¹ steht für Wasserstoff, eine C₁-C₄-Alkylgruppe oder eine C₁-C₄-Hydroxyalkylgruppe.
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Sälze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Mitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Bevorzugte erfindungsgemäße Mittel sind **dadurch gekennzeichnet, daß** sie mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Neben dem/den erfindungsgemäß enthaltenen beschichteten Alkalisierungsmittel(n) können die erfindungsgemäßen Mittel zur Nuancierung einen der mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43. Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Entsprechende erfindungsgemäße Mittel, die dadurch gekennzeichnet sind, daß sie mindestens einen direktziehenden Farbstoff aus der Gruppe der kationischen (basischen) Farbstoffe, vorzugsweise Basic Blue 6, C.I.-No. 51,175; Basic Blue 7. C.I. - No. 42,595; Basic Blue-9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74; C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23. C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. 1.-No. 45,425 : 1 (Solvent Red 73); D&C Orange No. 11, C.I.-No. 45,425 (Acid Red 95); D&C Red No. 21, C.I.-No: 45,380 : 2 (Solvent Red-43); D&C Red No. 27, C.I.-No. 45,410: 1 (Solvent Red 48); D8C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylhamstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, sind bevorzugte Ausführungsformen der vorliegenden Erfindung.

Unter den vorstehend genannten Farbstoffen sind einige Vertreter besonders bevorzugt, weshalb weiter bevorzugte erfindungsgemäße Mittel, die dadurch gekennzeichnet sind, daß sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse-Violet 4, HC Orange 1, HC Red 1, HC Red 3, HC Red 13, HC Yellow 2, HC Yellow 4, Na-Pikramat, 1,4-Bis-(2'-hydroxyethyt)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2'-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzot, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2,4-Nitrophenyl-aminoethylhamstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, bevorzugt sind.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

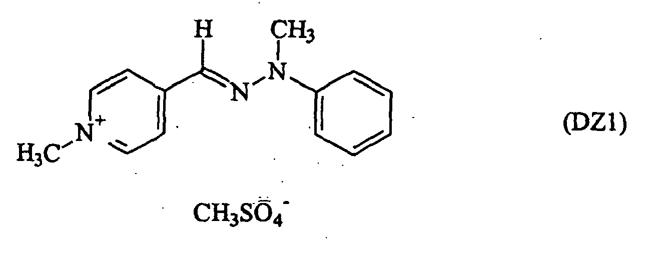

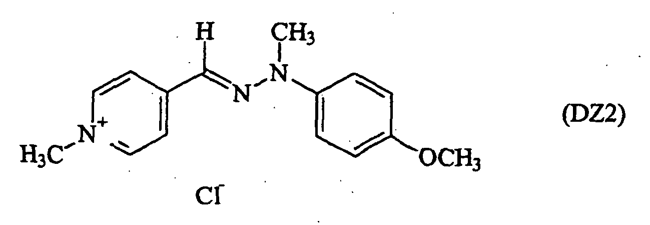

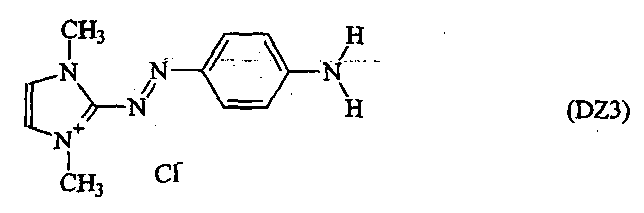

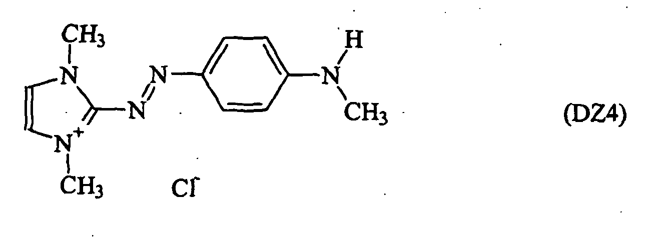

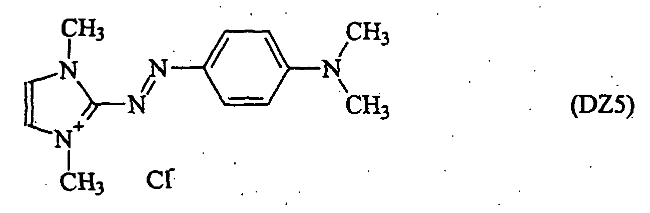

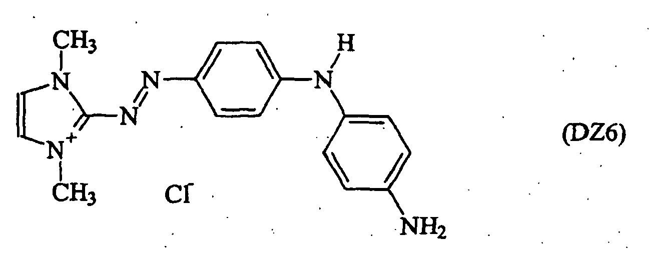

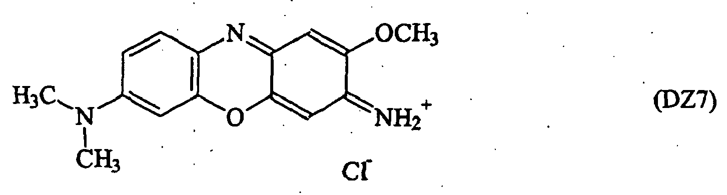

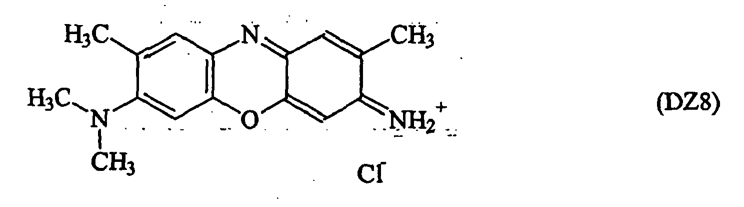

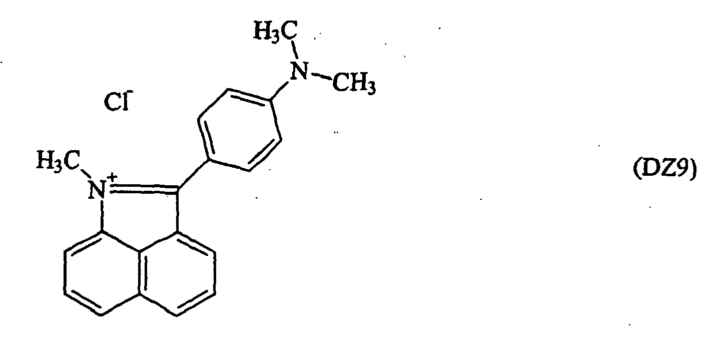

Die Verbindungen der Formeln (DZ1). (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Mittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

In einer weiteren Ausführungsform enthält das erfindungsgemäße Mittel zusätzlich eine Kombination aus Komponente
- A: Verbindungen, die eine reaktive Carbonylgruppe enthalten mit Komponente
- B: Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) von Alkoholen unter Bildung von Acetalen oder Ketalen als Kondensationsverbindung.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron,
Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3,-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylaminozimtaldehyd, 4-Dibutylamino-benzaldehyd, 4-Diphenylamino-benzaldehyd, 4-Dimethylamino-2-methoxybenzaldehyd, 4-(1-imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd,
1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on,
5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluoisulfonat, -methansulfonat, -perchlorat, -sulfat, -chlorid, -bromid, -iodid, -tetrachlorozinkat, -methylsulfat-, trifluormethansulfonat, -tetrafluoroborat,
lsatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quatemierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodidid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-mino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Die weiteren bevorzugten Verbindungen der Komponente B sind dem Fachmann aus der Druckschrift WO-A1-99/18916 (Seiten 6 bis 9) bekannt, auf die hier ausdrücklich und vollinhaltlich Bezug genommen wird.

Zusammenfassend sind erfindungsgemäße Mittel bevorzugt, die mindestens einen direktziehenden Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D& C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-Hydroxy-1,4-napthochinon; 1-Propen-(4-amino-2-nitrophenyl)amin; Isatin; N-methylylisatin; HC Violet 1; HC Violet 2; 4-Nitrophenyl-aminoethylhamstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Weiter bevorzugte erfindungsgemäße Mittel sind **dadurch gekennzeichnet, daß** sie mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylhamstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten.

Auch erfindungsgemäße Mittel, die mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthalten, sind erfindungsgemäß bevorzugt.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-lonen oder Komplexe dieser Ionen enthalten.

Bevorzugte erfindungsgemäße Haarfärbe- und - aufhellungsmittel enthalten 0,0001 bis 2,5 Gew.-%, vorzugsweise 0,001 bis 1 Gew.-%, bezogen auf die Gesamtzusammensetzung des Mittels, mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Kaliumjodid, Natriumjodid, Lithiumchlorid, Kaliumdichromat, Magnesiumacetat, Calciumchlorid, Calciumnitrat, Bariumnitrat, Mangandioxid (MnO₂) und/oder Hydrochinon.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Blondiermittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel I und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließende beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist ein Verfahren zur Herstellung von Mitteln zum Aufhellen und/oder Färben von Keratinfasern umfassend die Schritte
a) Beschichten mindestens eines Alkalisierungsmittels mit mindestens einem Komplexbilder;
b) Abmischen des/der beschichteten Alkalisierungsmittel(s) mit weiteren Inhaltstoffen von Blondier- und/Färbemitteln für keratinische Fasern.

Bezüglich bevorzugter Ausführungsformen des erfindungsgemäßen Verfahrens gilt hinsichtlich der Stoffauswahl mutatis mutandis das zu den erfindungsgemäßen Mitteln Gesagte.

So sind insbesondere erfindungsgemäße Verfahren bevorzugt, bei denen als Komplexbildner Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, eingesetzt werden.

In weiter bevorzugten erfindungsgemäßen Verfahren enthält das beschichtete Alkalisierungsmittel (inklusive Beschichtung), bezogen auf sein Gewicht, 5 bis 80 Gew.-%, vorzugsweise 10 bis 75 Gew.-% und insbesondere 20 bis 70 Ges.-% Alkalisierungsmittel.

Die Menge an Komplexbildner wird in weiter bevorzugten erfindungsgemäßen Verfahren so gewählt, daß das beschichtete Alkalisierungsmittel (inklusive Beschichtung), bezogen auf sein Gewicht, 0,1 bis 50 Gew.-%, vorzugsweise 3 bis 25 Gew.-% und insbesondere 5 bis 15 Gew.-% Komplexbildner enthält.

Im ersten Schritt des erfindungsgemäßen Verfahrens wird mindestens ein Alkalisierungsmittel mit mindestens einem Komplexbildner beschichtet. Dieser Schritt läßt sich in den unterschiedlichsten Apparaturen problemlos durchführen. In einer geeigneten Misch- und Granuliervorrichtung, beispielsweise in entsprechenden Anlagen vom Typ eines Eirich-Mischers, eines Lödige-Mischers, beispielsweise eines Pflugscharmischers der Firma Lödige, oder eines Mischers der Firma Schugi, kann bei Umfangsgeschwindigkeiten der Mischorgane vorzugsweise zwischen 2 und 7 m/s (Pflugscharmischer) beziehungsweise 3 bis 50 m/s (Eirich, Schugi), insbesondere zwischen 5 und 20 m/s ein Feststoffbett vorgelegt und nachfolgend unter Zusatz des feinteiligen Komplexbildners abgepudert werden. Dabei kann gleichzeitig in an sich bekannter Weise eine vorbestimmte Korngröße des Granulats eingestellt werden. Der Abpuderungsprozeß benötigt nur einen sehr kurzen Zeitraum von beispielsweise etwa 0,5 bis 10 Minuten, insbesondere etwa 0,5 bis 5 Minuten (Eirich-Mischer, Lödige-Mischer) zur Homogenisierung des Gemisches unter Ausbildung des rieselfähigen Granulates. Im Schugi-Mischer hingegen reicht normalerweise eine Verweilzeit von 0,5 bis 10 Sekunden aus, um ein rieselfähiges Granulat zu erhalten. Für die Durchführung dieses Verfahrensschritts geeignete Mischer sind beispielsweise Eirich^{®}-Mischer der Serien R oder RV (Warenzeichen der Maschinenfabrik Gustav Eirich, Hardheim), der Schugi^{®} Flexomix, die Fukae^{®} FS-G-Mischer (Warenzeichen der Fukae Powtech, Kogyo Co., Japan), die Lödige^{®} FM-. KM- und CB-Mischer (Warenzeichen der Lödige Maschinenbau GmbH, Paderborn) oder die Drais^{®}-Serien T oder K-T (Warenzeichen der Drais-Werke GmbH, Mannheim).

Es ist erfindungsgemäß auch möglich, mehrere der oben genannten Mischer in Reihe zu schalten. Insbesondere bieten sich hier folgende Kombinationen aufeinanderfolgender Mischer an:
- Lödige CB / Lödige KM
- Lödige KM / Schugi Flexomix
- Schugi Flexomix / Lödige KM / Schugi Flexomix
- Schugi Flexomix / Lödige CB
- Lödige CB / Lödige KM / Schugi Flexomix

Bevorzugte erfindungsgemäße Verfahren sind **dadurch gekennzeichnet, daß** Schritt a) in einem Mischergranulator durchgeführt wird, wobei das/die Alkalisierungsmittel in fester Form vorgelegt und mit feinteiligem Komplexbildner abgepudert wird/werden.

In weiter bevorzugten erfindungsgemäßen Verfahren wird zusätzlich eine Granulierflüssigkeit aufgegeben, wobei die Granulierflüssigkeit vorzugsweise frei von Tensid(en) und Komplexbildner(n) ist.

Alternativ zum Einsatz eines Mischergranulators kann auch eine Wirbelschichtapparatur zur Ausbildung des Feststoffcoatings genutzt werden. Erfindungsgemäße Verfahren, bei denen die Beschichtung des/der Alkalisierungsmittel in einer Wirbelschichtapparatur durchgeführt wird, sind bevorzugt.

Auch hier kann gleichzeitig Flüssigkeit auf die Körner aufgebracht werden. Die Beschichtung kann dabei gleichzeitig mit der Trocknung erfolgen (beispielsweise in einer Wirbelschichtapparatur, in der die Granulate mit einer Lösung oder Dispersion mindestens eines Komplexbildners beaufschlagt und gleichzeitig getrocknet werden), es ist aber auch möglich und bevorzugt, die Trocknung nach der Beschichtung, also zeitlich anschließend an diese, durchzuführen.

Alternativ zum Partikelcoating (Abpuderung) kann auch eine dichtere Hülle erzeugt werden, indem eine Lösung oder Dispersion des/der Komplexbildner(s) auf das/die. Alkalisierungsmittel aufgebracht wird. Diese Lösung bzw. Dispersion kann auch weitere filmbildende Substanzen enthalten. Ein erfindungsgemäßes Verfahren, bei dem das/die Alkalisierungsmittel mit einer Lösung oder Dispersion von Komplexbildner(n) besprüht und anschließend getrocknet wird/werden, ist demnach eine weitere bevorzugte Ausführungsform der vorliegenden Erfindung.

### Beispiele:

Folgende Rohstoffe wurden verwendet:
- ldranal^{®} III: Ethylendiamintetraessigsäure Dinatrium SAlz·2 H₂O (INCI-Bezeichnung: Disodium EDTA) (Hersteller: Riedel De Haen)
- Britesil® C20: Natriumsilikat (INCI-Bezeichnung: Sodium Silikate) (The PQ Corporation)

### 1.0 Herstellung des Kern-Hülle-Alkalisierungsmittels

Idranal^{®} III wurde zur Herstellung des Hüllmaterials in einer Fliehkraft-Kugelmühle (Fa. Retsch, Typ S 100) zunächst intensiv gemahlen (4 UpM, 4 Minuten). 5,0 g eines Gemisches aus Stearin-/Isostearinsäure (Gewichtsverhältnis der Säuren 1 : 3) wurde vorgelegt und auf ca. 65°C erwärmt. 9,0 g des gemahlenen Idranal^{®} III wurde homogen in das erwärmte Fettsäuregemisch eingearbeitet.

66,5 g Britesil^{®} C 20 wurden bei einer Temperatur von 65°C mit der gesamten oben erhaltenen homogenen Mischung überzogen. Das Gemisch wurde unter starkem Rühren auf Raumtemperatur abgekühlt.

Die Hülle des Alkalisierungsmittels enthielt 64 % Komplexbildner.

### 2.0 Wirksamkeitstest

Es wurden folgende Blondierpulverrezepturen nach bekanntem Verfahren hergestellt:

| | **E1** (erfindungsgemäß) | **V1** (Vergleich) |
|---|---|---|
| Ammoniumperoxidisulfat | 6,5 g | 6,5 g |
| Kaliumperoxidisulfat | 21,4 g | 21,4 g |
| Natriumperoxidisulfat | 7,8 g | 7,8 g |
| Kern-Hülle-Alkalisierungmittel | 16,1 g | - |
| Britesil^{®} C 20 | - | 13,3 g |
| Idranal^{®} III | - | 1,0 g |

Zu 50 g einer Entwicklerdispersion, enthaltend 12 Gew.% Wasserstoffperoxid (Poly^{®} Blond Ultra der Fa. Schwarzkopf & Henkel), wurden 20 g eines der oben genannten Blondierpulver gegeben und die Mischung in der Applikationsflasche des Handelsprodukts 30 Sekunden lang geschüttelt. Nach einer Ruhezeit von 20 Sekunden wurde die Applikationsflasche geöffnet und abgesteift. Ab Öffnen der Flasche wurde die Zeit bis zum selbsttätigen Austritt des Flascheninhalts aus der Flasche gemessen.

Blondierpulver E1: Es wurde keine Gasentwicklung beobachtet. Nach 2 Stunden wurde der Versuch abgebrochen, ohne dass das Mittel aus der Flasche austrat.

Blondierpulver V1: Es wurde eine Gasentwicklung beobachtet. Nach 1 Stunde und 38 Minuten trat der Flascheninhalt selbsttätig aus.

## Patentansprüche

1. Mittel zum Aufhellen und/oder Färben von Keratinfasern, insbesondre menschlichen Haaren, enthaltend mindestens ein festes Alkalisierungsmittel, **dadurch gekennzeichnet, daß** das Akalisierungsmittel einen Partikelkern umfaßt, der Alkalisierungsmittel enthält und eine diesen Kern umgebende Hülle aufweist, die zu mindestens 50 Gew.-%, vorzugsweise zu mindestens 70 Gew.-%, weiter bevorzugt zu mindestens 90 Gew.-% und insbesondere zu 100 Gew.-% ihres Gewichts aus Komplexbildner(n) besteht.

2. Mittel nach Anspruch 1, **dadurch gekennzeichnet, daß** die Hülle als Komplexbildner Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthält.

3. Mittel nach einem der Ansprüche 1 oder 2, **dadurch gekennzeichnet, daß** die Hülle neben Komplexbildner(n) Bindemittel enthält, wobei als Bindemittel natürliche Polymere, vorzugsweise Cellulose und/oder Stärke sowie deren Derivate, insbesondere Carboxymethylcellulose (CMC) und/oder Hydroxypropylcellulose (HPC) und/oder Hydroxypropylmethylcellulose (HPMC) bevorzugt sind und bevorzugte Mengen der Hülle an Bindemittel 1 bis 50 Gew.-%, vorzugsweise 5 bis 40 Gew.-% und insbesondere 10 bis 30 Gew.-% Bindemittel (jeweils bezogen auf die Beschichtung) betragen.

4. Mittel nach einem der Ansprüche 1 bis 3, **dadurch gekennzeichnet, daß** es zusätzlich eine feste Peroxoverbindung enthält, die vorzugsweise ausgewählt ist aus Wasserstoffperoxid-Anlagerungsverbindungen an feste Träger, Ammonium- und Alkalimetall-persulfaten und -peroxidisulfaten.

5. Mittel nach Anspruch 4, **dadurch gekennzeichnet, daß** es mindestens 2 verschiedene Peroxidisulfate enthält.

6. Mittel nach einem der Ansprüche 1 bis 5, **dadurch gekennzeichnet, daß** es mindestens einen direktziehenden Farbstoff aus der Gruppe der kationischen (basischen) Farbstoffe, vorzugsweise Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic. Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet 1, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 und/oder der anionischen (sauren) Farbstoffe, und/oder der nichtionischen Farbstoffe, vorzugsweise Acid Black 1, C.I.-No. 20,470; Acid Black 52; Acid Blue 7; Acid Blue 9, C.I.-No. 42,090; Acid Blue 74, C.I.-No. 73,015, Acid Red 18, C.I.-No. 16.255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No: 19,140; Acid Yellow 3, C.I.-No. 47,005; Acid Yellow 36; D&C Brown No. 1, C.I.-No. 20, 170 (Acid Orange 24); D&C Green No. 5, C.I.-No. 61,570 (Acid Green G); D&C Orange No. 4, C.I.-No. 15,510 (Acid Orange II); D&C Orange No. 10, C. I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C. I.-No. 45,350 : 1 (Solvent Yellow 94); D&C Yellow No. 8, C.I.-No. 45,350 (Acid Yellow 73); FD& C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na-Pikramat; 1,4-Bis-(2'-hydroxyethyl)amino- 2-nitro-p-phenylendiamin; HC Yellow 5; HC Blue 2; HC Blue 12; 4-Amino-3-nitrophenol; HC Yellow 6; HC Yellow 12; 2-Nitro-1-(2'hydroxyethyl)amino-4-methylbenzol; 2-Nitro-4-amino-diphenylamin-2-carbonsäure; 2-Amino-6-chlor-4-nitrophenol; HC Red BN; 6-Nitro-1,2,3,4-tetranitrochinoxalin; o-Nitro-p-phenylendiamin; p-Nitro-m-phenylendiamin; HC Red B 54; HC Red 10; HC Red 11; HC Red 13; 2-(2'-Hydroxyethyl)amino-1-hydroxy-4,6-dinitrobenzol; 4-Ethylamino-3-nitrobenzoesäure; 2-Chlor-6-ethylamino-4-nitrophenol; 2-HydroXy-1,4-napthochinon; 1-Propen-(4-amino-2-nitrophenyl)amin; Isatin; N-methylylisatin; HC Violet 1; HC Violet 2; 4-Nitrophenyl-aminoethylharnstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

7. Mittel nach einem der Ansprüche 1 bis 6, **dadurch gekennzeichnet, dass** es mindestens einen Direktzieher, ausgewählt aus Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na-Pikramat, 1,4-Bis-(2'-hydroxyethyl)amino-2-nitro- p-phenylendiamin, HC Yellow 5, HC Blue 2, HC Blue 12, 4-Amino-3-nitrophenol, HC Yellow 6, HC Yellow 12, 2-Nitro-1-(2'hydroxyethyl)amino-4-methylbenzol, 2-Nitro-4-amino-diphenylamin-2'-carbonsäure, 2-Amino-6-chlor-4-nitrophenol, HC Red BN; 6-Nitro-1,2,3,4-tetranitrochinoxalin, o-Nitro-p-phenylendiamin, p-Nitro-m-phenylendiamin, HC Red B 54, HC Red 10, HC Red 11, HC Red 13, 2-(2'-Hydroxyethyl)amino-1-hydroxy-4,6-dinitrobenzol, 4-Ethylamino-3-nitrobenzoesäure, 2-Chlor-6-ethylamino-4-nitrophenol, 2-Hydroxy-1,4-napthochinon, 1-Propen-(4-amino-2-nitrophenyl)amin, Isatin, N-Methylylisatin, HC Violet 1, HC Violet 2, 4-Nitrophenyl-aminoethylhamstoff in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

8. Mittel nach einem der Ansprüche 1 bis 7, **dadurch gekennzeichnet, daß** es mindestens einen Farbstoffvorläufer aus den Gruppen der aromatischen und heteroaromatischen Diamine, Aminophenole, Naphthole, Polyphenole CH-aciden Kupplerkomponenten und ihrer Derivate in Mengen von 0,01 bis 25 Gew.%, vorzugsweise von 0,5 bis 10 Gew.%, insbesondere von 1 bis 5 Gew.-%, jeweils bezogen auf das gesamte Mittel, enthält.

9. Verfahren zur Herstellung von Mitteln zum Aufhellen und/oder Färben von Keratinfasern, **gekennzeichnet durch** die Schritte
a) Beschichten mindestens eines Alkalisierungsmittets mit mindestens einem Komplexbildner;
b) Abmischen des/der beschichteten Alkalisierungsmittel(s) mit weiteren Inhaltstoffen von Blondier- und/Färbemitteln für keratinische Fasern.

10. Verfahren nach Anspruch 9, **dadurch gekennzeichnet, daß** als Komplexbildner Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, eingesetzt werden.

11. Verfahren nach einem der Ansprüche 9 oder 10, **dadurch gekennzeichnet, daß** das beschichtete Alkalisierungsmittel (inklusive Beschichtung), bezogen auf sein Gewicht, 5 bis 80 Gew.-%, vorzugsweise 10 bis 75 Gew.-% und insbesondere 20 bis 70 Gew.-% Alkalisierungsmittel enthält.

12. Verfahren nach einem der Ansprüche 9 bis 11, **dadurch gekennzeichnet, daß** das beschichtete Alkalisierungsmittel (inklusive Beschichtung), bezogen auf sein Gewicht, 0,1 bis 50 Gew.-%, vorzugsweise 3 bis 25 Gew.-% und insbesondere 5 bis 15 Gew.-% Komplexbildner enthält.

13. Verfahren nach einem der Ansprüche 9 bis 12, **dadurch gekennzeichnet, daß** Schritt a) in einem Mischergranulator durchgeführt wird, wobei das/die Alkalisierungsmittel in fester Form vorgelegt und mit feinteiligem Komplexbildner abgepudert wird/werden.

14. Verfahren nach Anspruch 13, **dadurch gekennzeichnet, daß** zusätzlich eine Granulierflüssigkeit aufgegeben wird, wobei die Granulierflüssigkeit vorzugsweise frei von Tensid(en) und Komplexbildner(n) ist.

15. Verfahren nach einem der Ansprüche 9 bis 12, **dadurch gekennzeichnet, daß** die Beschichtung des/der Alkalisierungsmittel in einer Wirbelschichtapparatur durchgeführt werden.

16. Verfahren nach einem der Ansprüche 9 bis 15, **dadurch gekennzeichnet, daß** das/die Alkalisierungsmittel mit einer Lösung oder Dispersion von Komplexbildner(n) besprüht und anschließend getrocknet wird/werden.

## Claims

1. An agent for lightening and/or coloring keratin fibers, in particular human hair, containing at least one solid alkalization agent, **characterized in that** the alkalization agent comprises a particle core, which contains alkalization agents and has a shell surrounding this core, which consists of at least 50% by weight, preferably at least 70% by weight, more preferably at least 90% by weight and in particular 100% by weight of complexing agent(s) based on its weight.

2. The agent according to claim 1, **characterized in that** the shell contains phosphonates, preferably hydroxyalkane or aminoalkane phosphonates and in particular 1-hydroxyethane-1,1-diphosphonate (HEDP) or its disodium or tetrasodium salts, and/or ethylenediamine-tetramethylene phosphonate (EDTMP) or its hexasodium salt and/or diethylenetriaminepentamethylene phosphonate (DTPMP) or its heptasodium or octasodium salt, as complexing agents.

3. The agent according to any of claims 1 or 2, **characterized in that** the shell contains in addition to complexing agent(s), binders, wherein natural polymers, preferably cellulose and/or starch, or their derivatives, in particular carboxymethylcellulose (CMC) and/or hydroxypropylcellulose (HPC) and/or hydroxypropylmethylcellulose (HPMC) are preferred as binders and preferred amounts of binders in the shell are from 1 to 50% by weight, preferably from 5 to 40% by weight and in particular from 10 to 30% by weight of binders (each based on the coating).

4. The agent according to any of claims 1 to 3, **characterized in that** it additionally contains a solid peroxo compound, which is preferably selected from hydrogen peroxide addition compounds on solid carriers, ammonium and alkali metal persulfates and peroxodisulfates.

5. The agent according to claim 4, **characterized in that** it contains at least two different peroxodisulfates.

6. The agent according to any of claims 1 to 5, **characterized in that** it contains at least one substantive dye from the group of cationic (basic) dyes, preferably Basic Blue 6, C.I.-No. 51,175; Basic Blue 7, C.I.-No. 42,595; Basic Blue 9, C.I.-No. 52,015; Basic Blue 26, C.I.-No. 44,045; Basic Blue 41, C.I.-No. 11,154; Basic Blue 99, C.I.-No. 56,059; Basic Brown 4, C.I.-No. 21,010; Basic Brown 16, C.I.-No. 12,250; Basic Brown 17, C.I.-No. 12,251; Basic Green 1, C.I.-No. 42,040; Basic Orange 31; Basic Red 2, C.I.-No. 50,240; Basic Red 22, C.I.-No. 11,055; Basic Red 46; Basic Red 51; Basic Red 76, C.I.-No. 12,245; Basic Violet, C.I.-No. 42,535; Basic Violet 2; Basic Violet 3, C.I.-No. 42,555; Basic Violet 10, C.I.-No. 45,170; Basic Violet 14, C.I.-No. 42,510; Basic Yellow 57, C.I.-No. 12,719; Basic Yellow 87 and/or of anionic (acid) dyes, and/or of non-ionic dyes, preferably Acid Black 1, C.I.-No. 20,470; Acid Black 52; Acid Blue 7; Acid Blue 9, C.I.-No. 42,090; Acid Blue 74, C.I.-No. 73,015; Acid Red 18, C.I.-No. 16,255; Acid Red 23; Acid Red 27, C.I.-No. 16,185; Acid Red 33; Acid Red 52; Acid Red 87, C.I.-No. 45,380; Acid Red 92, C.I.-No. 45,410; Acid Orange 3; Acid Orange 7; Acid Violet 43, C.I.-No. 60,730; Acid Yellow 1, C.I.-No. 10,316; Acid Yellow 10; Acid Yellow 23, C.I.-No. 19,140; Acid Yellow 3, C.I.-No. 47,005; Acid Yellow 36; D&C Brown No. 1, C.I.-No. 20,170 (Acid Orange 24); D&C Green No. 5, C.I.-No. 61,570 (Acid Green G); D&C Orange No. 4, C.I.-No. 15,510 (Acid Orange II); D&C Orange No. 10, C.I.-No. 45,425 : 1 (Solvent Red 73); D&C Orange No. 11, C.I.-No. 45,425 (Acid Red 95); D&C Red No. 21, C.I.-No. 45,380 : 2 (Solvent Red 43); D&C Red No. 27, C.I.-No. 45,410 : 1 (Solvent Red 48); D&C Red No. 33, C.I.-No. 17,200 (Acid Red 2A, Acid Red B); D&C Yellow No. 7, C.I.-No. 45,350 : 1 (Solvent Yellow 94); D&C Yellow No. 8, C.I.-No. 45,350 (Acid Yellow 73); FD&C Red No. 4, C.I.-No. 14,700 (Food Red 4); FD&C Yellow No. 6, C.I.-No. 15,985 (Food Yellow 3); Food Green 3; Pigment Red 57-1; Disperse Black 9; Disperse Blue 1; Disperse Blue 3; Disperse Violet 1; Disperse Violet 4; HC Orange 1; HC Red 1; HC Red 3; HC Red 13; HC Yellow 2; HC Yellow 4; Na picramate; 1,4-bis-(2'-hydroxyethyl)amino-2-nitro-p-phenylenediamine; HC Yellow 5; HC Blue 2; HC Blue 12; 4-amino-3-nitrophenol; HC Yellow 6; HC Yellow 12; 2-nitro-1-(2'-hydroxyethyl)amino-4-methylbenzene; 2-nitro-4-amino-diphenylamine-2-carboxylic acid; 2-amino-6-chloro-4-nitrophenol; HC Red BN; 6-nitro-1,2,3,4-tetranitroquinoxaline; o-nitro-p-phenylenediamine; p-nitro-m-phenylenediamine; HC Red B 54; HC Red 10; HC Red 11; HC Red 13; 2-(2'-hydroxyethyl)amino-1-hydroxy-4,6-dinitrobenzene; 4-ethylamino-3-nitrobenzoic acid; 2-chloro-6-ethylamino-4-nitrophenol; 2-hydroxy-1,4-naphthoquinone; 1-propene-(4-amino-2-nitrophenyl)amine; isatine; N-methylylisatine; HC Violet 1; HC Violet 2; 4-nitrophenyl-aminoethylurea in amounts from 0.01 to 25% by weight, preferably from 0.5 to 10% by weight, in particular from 1 to 5% by weight, each based on the total agent.

7. The agent according to any of claims 1 to 6, **characterized in that** it contains at least one substantive dye, selected from Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, Na picramate, 1,4-bis-(2'-hydroxyethyl)amino-2-nitro-p-phenylenediamine, HC Yellow 5, HC Blue 2, HC Blue 12, 4-amino-3-nitrophenol, HC Yellow 6, HC Yellow 12, 2-nitro-1-(2'-hydroxyethyl)amino-4-methylbenzene, 2-nitro-4-amino-diphenylamine-2-carboxylic acid, 2-amino-6-chloro-4-nitrophenol, HC Red BN; 6-nitro-1,2,3,4-tetranitroquinoxaline, o-nitro-p-phenylenediamine, p-nitro-n-phenylenediamine, HC Red B 54, HC Red 10, HC Red 11, HC Red 13, 2-(2'-hydroxyethyl)amino-1-hydroxy-4,6-dinitrobenzene, 4-ethylamino-3-nitrobenzoic acid, 2-chloro-6-ethylamino-4-nitrophenol, 2-hydroxy-1 ,4-naphthoquinone, 1-propene-(4-amino-2-nitrophenyl)-amine, isatine, N-methylylisatine, HC Violet 1, HC Violet 2, 4-nitrophenyl-aminoethylurea in amounts from 0.01 to 25% by weight, preferably from 0.5 to 10% by weight, in particular from 1 to 5% by weight, each based on the total agent.

8. The agent according to any of claims 1 to 7, **characterized in that** it contains at least a dye precursor from the groups of aromatic and heteroaromatic diamines, aminophenols, naphthols, polyphenols, CH-acid coupling components and their derivatives in amounts from 0.01 to 25% by weight, preferably from 0.5 to 10% by weight, in particular from 1 to 5% by weight, each based on the total agent.

9. A method for preparing agents for lightening and/or coloring keratin fibers, **characterized by** the steps
a) coating at least one alkalization agent with at least one complexing agent;
b) mixing the coated alkalization agent(s) with further ingredients of blond dying agents and dyes for keratin fibers.

10. The method according to claim 9, **characterized in that**, phosphonates, preferably hydroxyalkane or aminoalkane phosphonates, and in particular 1-hydroxyethane-1,1-diphosphonate (HEDP) or its disodium or tetrasodium salt, and/or ethylenediaminetetramethylene phosphonate (EDTMP) or its hexasodium salt, and/or diethylenetriaminepentamethylene phosphonate (DTPMP) or its heptasodium or octasodium salt, are applied as complexing agents.

11. The method according to any of claims 9 or 10, **characterized in that** the coated alkalization agent (coating included) contains, based on its weight, 5 to 80% by weight, preferably 10 to 75% by weight, and in particular 20 to 70% by weight of alkalization agent.

12. The method according to any of claims 9 to 11, **characterized in that** the coated alkalization agent (coating included), contains, based on its weight, 0.1 to 50% by weight, preferably 3 to 25% by weight and in particular 5 to 15% by weight of complexing agent.

13. The method according to any of claims 9 to 12, **characterized in that** step a) is carried out in a mixer-granulator, wherein the alkalization agent(s) is (are) placed in solid form and powdered with finely divided complexing agent.

14. The method according to claim 13, **characterized in that** a granulating liquid is added, wherein the granulating liquid is preferably free of surfactant(s) and of complexing agent(s).

15. The method according to any of claims 9 to 12, **characterized in that** the coating of the alkalization agent(s) is carried out in a fluidized bed apparatus.

16. The method according to any of claims 9 to 15, **characterized in that** the alkalization agent(s) is sprayed with a solution or dispersion of complexing agent(s) and subsequently dried.

## Revendications

1. Agent pour la décoloration et/ou la coloration de fibres kératiniques, en particulier de cheveux humains contenant au moins un agent d'alcalinisation solide, **caractérisé en ce que** l'agent d'alcalinisation comprend un noyau particulaire qui contient un ou plusieurs agents d'alcalinisation et présente une enveloppe entourant ce noyau, qui est constituée à concurrence d'au moins 50 % en poids, de préférence à concurrence d'au moins 70 % en poids, de manière plus préférée à concurrence d'au moins 90 % en poids et en particulier à concurrence de 100 % en poids de son poids, par un ou plusieurs agents complexants.

2. Agent selon la revendication 1, **caractérisé en ce que** l'enveloppe contient, à titre de agents complexants, des phosphonates, de préférence des phosphonates d'hydroxyalcanes, respectivement d'aminoalcanes et en particulier le 1-hydroxyéthane-1,1-diphosphonate (HEDP), respectivement son sel disodique ou tétrasodique et/ou l'éthylènediaminetétraméthylènephosphonate (EDTMP), respectivement son sel hexasodique et/ou le diéthylènetriaminepentaméthylènephosphonate (DTPMP), respectivement son sel heptasodique ou octasodique.

3. Agent selon la revendication 1 ou 2, **caractérisé en ce que** l'enveloppe contient, à côté du ou des agents complexants, des liants, des liants préférés étant des polymères naturels, de préférence de la cellulose et/ou de l'amidon ainsi que leurs dérivés, en particulier la carboxyméthylcellulose (CMC) et/ou l'hydroxypropylcellulose (HPC) et/ou l'hydroxypropylméthylcellulose (HPMC), et des quantités préférées des liants dans l'enveloppe s'élevant de 1 à 50 % en poids, de préférence de 5 à 40 % en poids et en particulier de 10 à 30 % en poids de liant (chaque fois rapportés à l'enrobage).

4. Agent selon l'une quelconque des revendications 1 à 3, **caractérisé en ce qu'**il contient, en outre, un composé peroxo solide, qui est choisi de préférence parmi des composés d'addition de peroxyde d'hydrogène sur des supports solides, des persulfates et des peroxodisulfates d'ammonium et de métaux alcalins.

5. Agent selon la revendication 4, **caractérisé en ce qu'**il contient au moins 2 peroxodisulfates différents.

6. Agent selon l'une quelconque des revendications 1 à 5, **caractérisé en ce qu'**il contient au moins un colorant direct, choisi parmi le groupe des colorants cationiques (basiques), de préférence Basic Blue 6, C.I.-N° 51,175 ; Basic Blue 7, CA.-N° 42,595 ; Basic Blue 9, C.I.-N° 52,015 ; Basic Blue 26, C.I.-N° 44,045 ; Basic Blue 41, C.I.-N° 11,154 ; Basic Blue 99, CA.-N° 56,059 ; Basic Brown 4, CA.-N° 21,010 ; Basic Brown 16, C.I.-N° 12,250 ; Basic Brown 17, C.I.-N° 12,251 ; Basic Green 1, C.I.-N° 42,040 ; Basic Orange 31 ; Basic Red 2, C.I.-N° 50,240 ; Basic Red 22, CA.-N° 11,055; Basic Red 46; Basic Red 51 ; Basic Red 76, CA.-N° 12,245 ; Basic Violet 1, C.I.-N° 42,535 ; Basic Violet 2 ; Basic Violet 3, CA.-N° 42,555 ; Basic Violet 10, C.I.-N° 45,170 ; Basic Violet 14, C.I.-N° 42,510; Basic Yellow 57, C.I.-N° 12,719; Basic Yellow 87 et/ou des colorants anioniques (acides), et/ou des colorants non ioniques, de préférence Acid Black 1, C.I.- N° 20,470 ; Acid Black 52 ; Acid Blue 7 ; Acid Blue 9, C.I.-N° 42,090 ; Acid Blue 74, C.I.-N° 73,015 ; Acid Red 18, CA.-N° 16,255 ; Acid Red 23 ; Acid Red 27, CA.-N° 16,185 ; Acid Red 33 ; Acid Red 52 ; Acid Red 87, CA.-N° 45,380 ; Acid Red 92, CA.-N° 45,410 ; Acid Orange 3 ; Acid Orange 7 ; Acid Violet 43, C.I.-N° 60,730 ; Acid Yellow 1, CA.-N° 10,316 ; Acid Yellow 10 ; Acid Yellow 23, C.I.-N° 19,140 ; Acid Yellow 3, C.I.-N° 47,005 ; Acid Yellow 36 ; D&C Brown N° 1. C.I.-N° 20,170 (Acid Orange 24); D&C Green N° 5, C.I.-N° 61,570 (Acid Green G) ; D&C Orange N° 4, C.I.-N° 15,510 (Acid Orange II); D&C Orange N° 10, C.I-N° 45,425 : 1 (Solvent Red 73); D&C Orange N° 11, C.I.-N° 45,425 (Acid Red 95) ; D&C Red N° 21, CA.-N° 45,380 : 2 (Solvent Red 43) ; D&C Red N° 27, C.I.-N° 45,410 : 1 (Solvent Red 48) ; D&C Red N° 33, C.I.-N° 17,200 (Acid Red 2A, Acid Red B) ; D&C Yellow N° 7, C.I.-N° 45,350 : 1 (Solvent Yellow 94) ; D&C Yellow N° 8, CA.-N° 45,350 (Acid Yellow 73) ; FD&C Red N° 4, C.I.-N° 14,700 (Food Red 4) ; FD&C Yellow N° 6, C.I.-N° 15,985 (Food Yellow 3); Food Green 3; Pigment Red 57-1 ; Disperse Black 9 ; Disperse Blue 1 ; Disperse Blue 3 ; Disperse Violet 1 ; Disperse Violet 4 ; HC Orange 1 ; HC Red 1 ; HC Red 3; HC Red 13; HC Yellow 2; HC Yellow 4; le picramate de sodium ; la 1,4-bis-(2'-hydroxyéthyl)amino-2-nitro-p-phénylèndiamine ; HC Yellow 5 ; HC Blue 2 ; HC Blue 12 ; le 4-amino-3-nitrophénol ; HC Yellow 6 ; HC Yellow 12 ; le 2-nitro-1-(2'-hydroxyéthyl)amino-4-méthylbenzène ; l'acide 2-nitro-4-amino-diphénylamine-2-carboxylique ; le 2-amino-6-chloro-4-nitrophénol ; HC Red BN ; la 6-nitro-1,2,3,4-tétranitroquinoxaline ; la o-nitro-p-phénylènediamine ; la p-nitro-m-phénylènediamine ; HC Red B 54 ; HC Red 10 ; HC Red 11 ; HC Red 13 ; le 2-(2'-hydroxyéthyl)amino-1-hydroxy-4,6-dinitrobenzène ; l'acide 4-éthylamino-3-nitrobenzoïque ; le 2-chloro-6-éthylamino-4-nitrophénol ; la 2-hydroxy-1,4-naphtoquinone ; la 1-propén-(4-amino-2-nitrophényl)amine ; l'isatine ; la N-méthylylisatine ; HC Violet 1 ; HC Violet 2 ; la 4-nitrophényl-aminoéthylurée, dans des quantités de 0,01 à 25 % en poids, de préférence de 0,5 à 10 % en poids, en particulier de 1 à 5 % en poids, chaque fois rapportés à l'agent dans sa totalité.

7. Agent selon l'une quelconque des revendications 1 à 6, **caractérisé en ce qu'**il contient au moins un colorant direct, choisi parmi Basic Blue 7, Basic Blue 99, Basic Violet 14, Basic Brown 16, Basic Brown 17, Basic Orange 31, Basic Red 46, Basic Red 51, Basic Red 76, Basic Yellow 57, Basic Yellow 87, Acid Black 1, Acid Blue 7, Acid Violet 43, Acid Red 23, Acid Red 52, Acid Orange 7, Acid Yellow 1, Acid Yellow 10, Acid Yellow 36, Food Green 3, Pigment Red 57-1, Disperse Black 9, Disperse Blue 1, Disperse Blue 3, Disperse Violet 1, Disperse Violet 4, HC Orange 1, HC Red 1, HC Red 3, HC Red 13, HC Yellow 2, HC Yellow 4, le picramate de sodium, la 1,4-bis-(2'-hydroxyéthyl)amino-2-nitro-p-phénylèndiamine, HC Yellow 5, HC Blue 2, HC Blue 12, le 4-amino-3-nitrophénol, HC Yellow 6, HC Yellow 12, le 2-nitro-1-(2'-hydroxyéthyl)amino-4-méthylbenzène, l'acide 2-nitro-4-amino-diphénylamine-2-carboxylique, le 2-amino-6-chloro-4-nitrophénol, HC Red BN; la 6-nitro-1,2,3,4-tétranitroquinoxaline, la o-nitro-p-phénylènediamine, la p-nitro-m-phénylènediamine, HC Red B 54, HC Red 10, HC Red 11, HC Red 13, le 2-(2'-hydroxyéthyl)amino-1-hydroxy-4,6-dinitrobenzène, l'acide 4-éthylamino-3-nitrobenzoïque, le 2-chloro-6-éthylamino-4-nitrophénol, la 2-hydroxy-1,4-naphtoquinone, la 1-propén-(4-amino-2-nitrophényl)amine, l'isatine, la N-méthylylisatine, HC Violet 1, HC Violet 2, la 4-nitrophénylaminoéthylurée, dans des quantités de 0,01 à 25 % en poids, de préférence de 0,5 à 10 % en poids, en particulier de 1 à 5 % en poids, chaque fois rapportés à l'agent dans sa totalité.

8. Agent selon l'une quelconque des revendications 1 à 7, **caractérisé en ce qu'**il contient au moins un précurseur de colorant choisi parmi les groupes des diamines aromatiques et hétéroaromatiques, des aminophénols, des naphtols, des polyphénols, des composants faisant office de coupleurs contenant un ou plusieurs groupes CH-acides et leurs dérivés, dans des quantités de 0,01 à 25 % en poids, de préférence de 0,5 à 10 % en poids, en particulier de 1 à 5 % en poids, chaque fois rapportés à l'agent dans sa totalité.

9. Procédé pour la préparation d'agents pour la décoloration et/ou la coloration de fibres kératiniques, **caractérisé par** les étapes
a) d'enrobage d'au moins un agent d'alcalinisation avec au moins un agent complexant ;
b) de mélange de l'agent/des agents d'alcalinisation enrobés avec d'autres constituants d'agents de décoloration et/ou de coloration pour des fibres kératiniques.

10. Procédé selon la revendication 9, **caractérisé en ce qu'**on met en oeuvre, à titre d'agents complexants, des phosphonates, de préférence des phosphonates d'hydroxyalcanes, respectivement d'aminoalcanes et en particulier le 1-hydroxyéthane-1,1-diphosphonate (HEDP), respectivement son sel disodique ou tétrasodique et/ou l'éthylènediaminetétraméthylènephosphonate (EDTMP), respectivement son sel hexasodique et/ou le diéthylènetriaminepentaméthylènephosphonate (DTPMP), respectivement son sel heptasodique ou octasodique.

11. Procédé selon l'une quelconque des revendications 9 ou 10, **caractérisé en ce que** l'agent d'alcalinisation enrobé contient (enrobage inclus), rapportés à son poids, un ou plusieurs agents d'alcalinisation à concurrence de 5 à 80 % en poids, de préférence à concurrence de 10 à 75 % en poids et en particulier à concurrence de 20 à 70 % en poids.

12. Procédé selon l'une quelconque des revendications 9 à 11, **caractérisé en ce que** l'agent d'alcalinisation enrobé contient (enrobage inclus), rapportés à son poids, un ou plusieurs agents complexants à concurrence de 0,1 à 50 % en poids, de préférence à concurrence de 3 à 25 % en poids et en particulier à concurrence de 5 à 15 % en poids.

13. Procédé selon l'une quelconque des revendications 9 à 12, **caractérisé en ce qu'**on effectue l'étape a) dans un mélangeur/granulateur, l'agent/des agents d'alcalinisation étant déposés au préalable sous forme solide et étant recouverts par saupoudrage avec un ou plusieurs agents complexants finement divisés.

14. Procédé selon la revendication 13, **caractérisé en ce qu'**on applique en outre un liquide de granulation, le liquide de granulation étant de préférence exempt d'agent(s) tensioactif(s) et d'agent(s) complexant(s).

15. Procédé selon l'une quelconque des revendications 9 à 12, **caractérisé en ce que** l'enrobage de l'agent/des agents d'alcalinisation a lieu dans un appareil à lit fluidisé.

16. Procédé selon l'une quelconque des revendications 9 à 15, **caractérisé en ce que** l'agent/les agents d'alcalinisation est/sont recouvert(s) par pulvérisation avec une solution ou une dispersion d'agent(s) complexant(s) et ensuite est/sont séché(s).
